(19) **Europäisches Patentamt / European Patent Office / Office européen des brevets**

(11) **EP 1 852 090 B1**

(12) **EUROPEAN PATENT SPECIFICATION**

(45) Date of publication and mention
of the grant of the patent:
**14.01.2009 Bulletin 2009/03**

(51) Int Cl.:
**A61F 2/16** (2006.01)

(21) Application number: **07106969.4**

(22) Date of filing: **25.04.2007**

(54) **Accommodative intraocular lens system**

Akkomodatives Intraokularlinsensystem

Système de lentille intraoculaire d'accommodation

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **02.05.2006 US 415906**

(43) Date of publication of application:
**07.11.2007 Bulletin 2007/45**

(73) Proprietor: **Alcon, Inc.
6331 Hünenberg (CH)**

(72) Inventor: **Tran, Son Trung
Arlington, TX 76017 (US)**

(74) Representative: **Hanna, Peter William Derek et al
Hanna Moore & Curley
13 Lower Lad Lane
Dublin 2 (IE)**

(56) References cited:
**WO-A-03/059196          US-A- 6 013 101
US-A1- 2003 204 254     US-A1- 2005 125 059
US-B1- 7 018 410**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Background of the Invention

This invention relates generally to the field of intraocular lenses (IOL) and, more particularly, to accommodative IOLs.

The human eye in its simplest terms functions to provide vision by transmitting light through a clear outer portion called the cornea, and focusing the image by way of a crystalline lens onto a retina. The quality of the focused image depends on many factors including the size and shape of the eye, and the transparency of the cornea and the lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, an opening is made in the anterior capsule and a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is replaced by an artificial lens.

In the natural lens, bifocality of distance and near vision is provided by a mechanism known as accommodation. The natural lens, early in life, is soft and contained within the capsular bag. The bag is suspended from the ciliary muscle by the zonules. Relaxation of the ciliary muscle tightens the zonules, and stretches the capsular bag. As a result, the natural lens tends to flatten. Tightening of the ciliary muscle relaxes the tension on the zonules, allowing the capsular bag and the natural lens to assume a more rounded shape. In the way, the natural lens can be focus alternatively on near and far objects.

As the lens ages, it becomes harder and is less able to change shape in reaction to the tightening of the ciliary muscle. This makes it harder for the lens to focus on near objects, a medical condition known as presbyopia. Presbyopia affects nearly all adults over the age of 45 or 50.

Prior to the present invention, when a cataract or other disease required the removal of the natural lens and replacement with an artificial IOL, the IOL was a monofocal lens, requiring that the patient use a pair of spectacles or contact lenses for near vision. Advanced Medical Optics has been selling a bifocal IOL, the Array lens, for several years, but due to quality of issues, this lens has not been widely accepted.

Several designs for accommodative IOLs are being studied. For example, several designs manufactured by C&C Vision are currently undergoing clinical trials. See U.S. Patent Nos. 6,197,059, 5,674,282, 5,496,366 and 5,476,514 (Cumming). The lens described in these patents is a single optic lens having flexible haptics that allows the optic to move forward and backward in reaction to movement of the ciliary muscle. A similar designs are described in U.S. Patent No. 6,302,911 B1 (Hanna), 6,261,321 B1 and 6,241,777 B1 (both to Kellan). The amount of movement of the optic in these single-lens systems, however, may be insufficient to allow for a useful range of accommodation. In addition, as described in U.S. Patent Nos. 6,197,059, 5,674,282, 5,496,366 and 5,476,514, the eye must be paralyzed for one to two weeks in order for capsular fibrosis to entrap the lens that thereby provide for a rigid association between the lens and the capsular bag. In addition, the commercial models of these lenses are made from a hydrogel or silicone material. Such materials are not inherently resistive to the formation of posterior capsule opacification ("PCO"). The only treatment for PCO is a capsulotomy using a Nd:YAG laser that vaporizes a portion of the posterior capsule. Such destruction of the posterior capsule may destroy the mechanism of accommodation of these lenses.

There have been some attempts to make a two-optic accommodative lens system. For example, U.S. Patent No. 5,275,623 (Sarfarazi), WIPO Publication No. 00/66037 (Glick, et al.) and WO 01134067 A1 (Bandhauer, et al), all disclose a two-optic lens system with one optic having a positive power and the other optic having a negative power. The optics are connected by a hinge mechanism that reacts to movement of the ciliary muscle to move the optics closer together or further apart, thereby providing accommodation. In order to provide this "zoom lens" effect, movement of the ciliary muscle must be adequately transmitted to the lens system through the capsular bag, and none of these references disclose a mechanism for ensuring that there is a tight connection between the capsular bag and the lens system. In addition, none of these lenses designs have addressed the problem with PCO noted above.

US 2003/204254 (Alcon Research) and US 7,018,410 (Vazeen) describe accommodative IOL systems in which an optic is captively locked to a ring-like element by flexible arms or haptics.

Therefore, a need continues to exist for a safe and stable accommodative intraocular lens system that provides accommodation over a broad and useful range.

Brief Summary of the Invention

The present invention improves upon the prior art by providing a two-optic accommodative lens system, in accordance with claims which follow. The first lens has a negative power and is located posteriorly within the capsular bag and lying against the posterior capsule. The periphery of the first lens is attached to a ring-like structure having a side wall. The second lens is located anteriorly to the first lens within of the capsular bag and is of a positive power. The peripheral edge of the second lens contains a plurality of haptics that are arranged in a spiral pattern and project posteriorly from the second lens and toward the first lens. The haptics are relatively firm, yet still flexible and ride within the side wall of the ring-like structure, so that flattening or steepening of the capsule in reaction to movement of the ciliary muscle and causes the second lens to move along the optical axis of the lens system.

Accordingly, one objective of the present invention is to provide a safe and biocompatible intraocular lens.

Another objective of the present invention is to provide a safe and biocompatible intraocular lens that is easily implanted in the posterior chamber.

Still another objective of the present invention is to provide a safe and biocompatible intraocular lens that is stable in the posterior chamber.

Still another objective of the present invention is to provide a safe and biocompatible accommodative lens system.

These and other advantages and objectives of the present invention will become apparent from the detailed description and claims that follow.

Brief Description of the Drawing

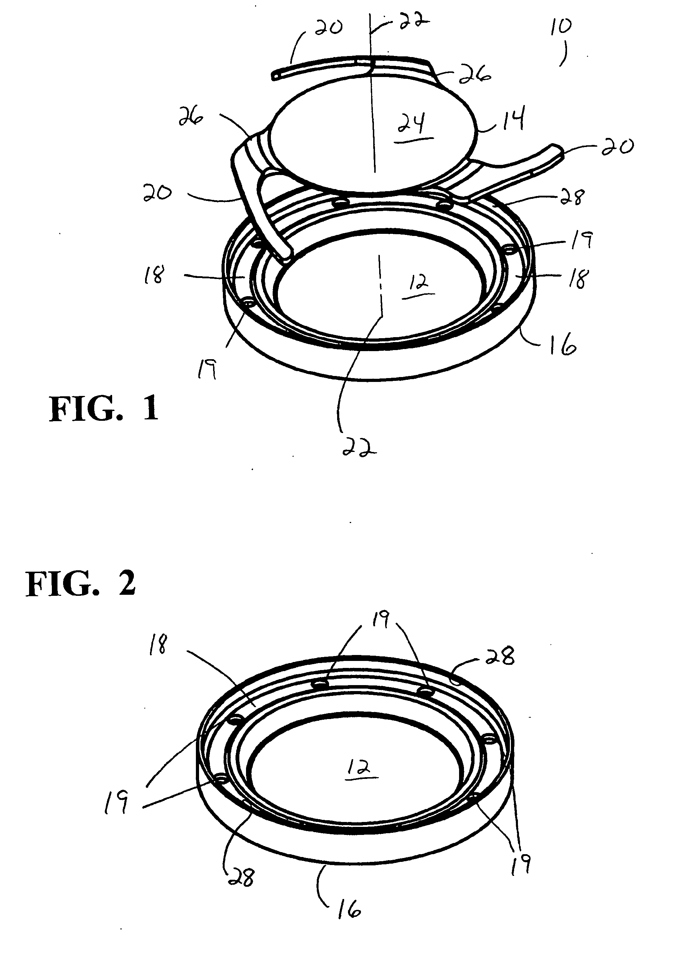

FIG. 1 is an enlarged, exploded perspective view of the lens system of the present invention.
FIG. 2 is an enlarged perspective view of the first lens of the lens system of the present invention.

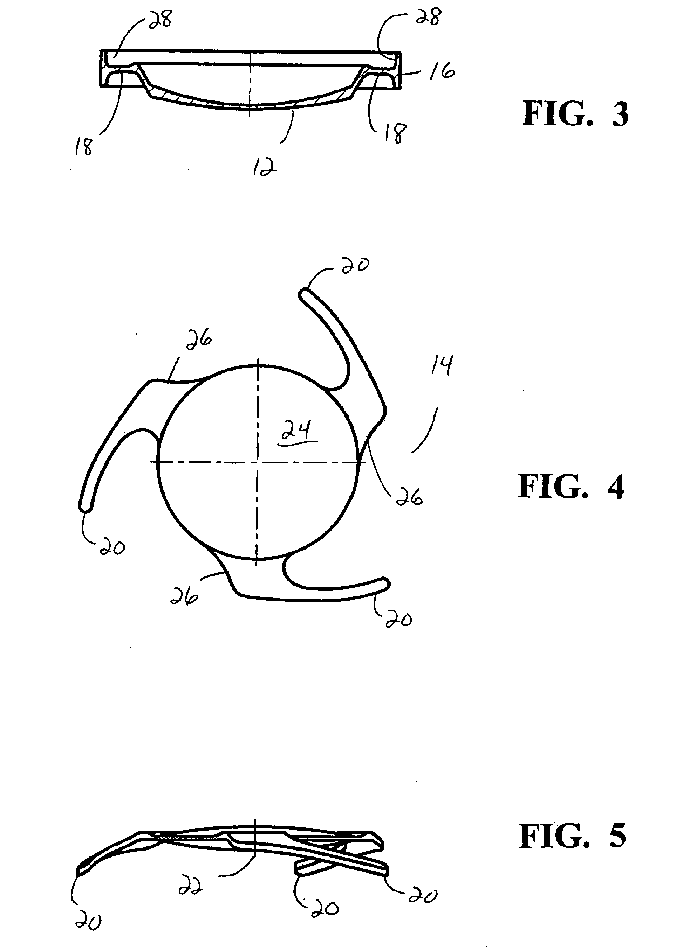

FIG. 3 is an enlarged cross-sectional view of the first lens of the lens system of the present invention.
FIG. 4 is an enlarged top plan view of the second lens of the lens system of the present invention.
FIG. 5 is an enlarged elevational view of the second lens of the lens system of the present invention.

Detailed Description of the Invention

As best seen in the figures, lens system 10 of the present invention generally consists of posterior lens 12, anterior lens 14 and circumferential ring 16. Lens 12 is preferably integrally formed with ring 16. Lens 12 preferably is made from a soft, foldable material that is inherently resistive to the formation of PCO, such as a soft acrylic. Lens 14 preferable is made from a soft, foldable material such as a hydrogel, silicone or soft acrylic. Lens 12 may be any suitable power, but preferably has a negative power. Lens 14 may also be any suitable power but preferably has a positive power. The relative powers of lenses 12 and 14 should be such that the axial movement of lens 14 toward or away from lens 12 should be sufficient to adjust the overall power of lens system 10 at least one diopter and preferably, at least three to four diopters, calculation of such powers of lenses 12 and 14 being within the capabilities of one skilled in the art of designing ophthalmic lenses by, for example, using the following equations:

$$P = P_1 + P_2 - T/n * P_1P_2 \qquad (1)$$

$$\delta P = -\delta T/n * P_1P_2 \qquad (2)$$

Lens 12 is generally symmetrical about optical axis 22. Peripheral band 18 connects lens 12 with ring 16 is relatively stiff, so as to allow some, but not excessive, flexing in response to ciliary muscle contraction and relaxation. Peripheral band 18 may contain a plurality of holes 19 for allowing the release or aspiration of any viscoelastic material used during surgery from behind optic 12 and/or band 18. Ring 16 has generally upright sidewall 28 that project anteriorly. Lens 14 contains a plurality of haptics 20 that project outward from optic 24 of lens 14 and away from optic 24 of lens 14 along optical axis 22. Second flexible haptics 20 are connected to lens 14 by regions 26 that are relatively stiff and allow second haptics 20 to exhibit resistive, spring-like movement. When compressed, second haptics 20 store energy, releasing that energy when uncompressed. Regions 26 also help create a space between the anterior capsule (not shown) and optic 24 that allow fluid to flow between the posterior and anterior sides of optic 24.

In use, lens 12 is implanted into the capsular bag prior to the implantation of lens 14. Lens 12 is held within the capsular bag by ring 16. Lens 14 is implanted so that second haptics 20 ride within sidewall 28 of ring 16. Lenses 12 and 14 are free-floating and not connected to each other. Upon implantation of lens 14, haptics 20 will flex in response to flattening and steepening of the lens capsule resulting from contraction and relaxation of the ciliary muscles. Flattening of the capsule caused by relaxation of the ciliary muscles will compress haptics 20 axially and allow second optic 24 to move posteriorly along optical axis 22 toward lens 12. Reduction of zonular tension caused by contraction of the ciliary muscles will allow energy stored in compressed haptics 20 to release, allowing optic 24 of lens 14 to move anteriorly along optical axis 22 and away from lens 12 because of the vaulted and spiral arrangement of haptics 20.

This description is given for purposes of illustration and explanation. It will be apparent to those skilled in the relevant art that changes and modifications may be made to the invention described above without departing from its scope.

**Claims**

1. An accommodative intraocular lens system (10), comprising:

   a first lens (12);
   a second lens (14) adapted to be arranged relative to the first lens so as to be movable along the optical axis (22) of the system,
   a ring (16) attached to the first lens (12), the ring having a sidewall (28) that projects anteriorly; and
   wherein the second lens (14) comprises a plurality of haptics (20) adapted to vault anteriorly and to fit within said sidewall (28),

   **characterized in that**
   the haptics are adapted to vault spirally from the second lens and are adapted to ride within the sidewall of the ring.

2. The lens of claim 1, wherein the haptics (20) are attached to the second lens (14) by hinge regions (26) that allow the second lens to move away from the first lens (12) in response to compression of the ring (16).

3. The lens of claim 1 or claim 2, wherein the first lens (12) is attached to the ring (16) by a peripheral band (18).

4. The lens of claim 3, wherein the peripheral band (18) comprises a plurality of holes (19).

5. The lens of any one of claims 1 to 4, wherein the first lens (12) and the second lens (14) comprise a soft acrylic material.

6. The lens of any one of claims 1 to 4, wherein the second lens (14) comprises a hydrogel material.

7. The lens of any one of claims 1 to 4, wherein the second lens (14) comprises a silicone material.

**Patentansprüche**

1. Akkomodations-Intraokular-Linsensystem (10), welches enthält:

   eine erste Linse (12);
   eine zweite Linse (14), welche dazu ausgelegt ist, in Relation zu der ersten Linse angeordnet zu werden, um somit entlang der optischen Achse (22) von dem System bewegbar zu sein;
   einen Ring (16), welcher an der ersten Linse (12) befestigt ist, wobei der Ring eine Seitenwand (28) hat, welche nach vorne vorragt; und
   wobei die zweite Linse (14) eine Mehrzahl von Haptiken (20) enthält, welche dazu ausgelegt sind, nach vorne überzuwölben und innerhalb der Seitenwand (28) einzupassen,
   **dadurch gekennzeichnet, dass**
   die Haptiken dazu ausgelegt sind, spiralförmig von der zweiten Linse überzuwölben, und dazu ausgelegt sind, innerhalb der Seitenwand von dem Ring zu verlaufen.

2. Linse nach Anspruch 1, bei welcher die Haptiken (20) durch Gelenkbereiche (26) an der zweiten Linse (14) befestigt sind, welche eine Bewegung der zweiten Linse von der ersten Linse (12) weg in Ansprechen auf eine Komprimierung von dem Ring (16) erlauben.

**3.** Linse nach Anspruch 1 oder 2, bei welcher die erste Linse (12) durch ein peripheres Band (18) an dem Ring (16) befestigt ist.

**4.** Linse nach Anspruch 3, bei welcher das periphere Band (18) eine Mehrzahl von Löchern (19) enthält.

**5.** Linse nach einem der Ansprüche 1 bis 4, bei welcher die erste Linse (12) und die zweite Linse (14) ein nachgiebiges Acrylmaterial enthalten.

**6.** Linse nach einem der Ansprüche 1 bis 4, bei welcher die zweite Linse (14) ein Hydrogelmaterial enthält.

**7.** Linse nach einem der Ansprüche 1 bis 4, bei welcher die zweite Linse (14) ein Silikonmaterial enthält.

**Revendications**

**1.** Système de lentille intraoculaire accommodative (10), comprenant :

une première lentille (12),
une seconde lentille (14) adaptée pour être agencée par rapport à la première lentille de manière à être mobile le long de l'axe optique (22) du système,
un anneau (16) fixé sur la première lentille (12), l'anneau ayant une paroi latérale (28) qui fait saillie antérieurement, et
dans lequel la seconde lentille (14) comporte une pluralité d'éléments haptiques (20) adaptés pour être bombés antérieurement, et pour s'agencer dans ladite paroi latérale (28),
**caractérisé en ce que**
les éléments haptiques sont adaptés pour être bombés en spirale par rapport à la seconde lentille, et sont adaptés pour un chevauchement dans la paroi latérale de l'anneau.

**2.** Lentille selon la revendication 1, dans laquelle les éléments haptiques (20) sont fixés sur la seconde lentille (14) par des zones d'articulation (26) qui permettent à la seconde lentille de se déplacer loin de la première lentille (12) en réponse à une compression de l'anneau (16).

**3.** Lentille selon la revendication 1 ou 2, dans laquelle la première lentille (12) est fixée sur l'anneau (16) par une bande périphérique (18).

**4.** Lentille selon la revendication 3, dans lequel la bande périphérique (18) comprend une pluralité de trous (19).

**5.** Lentille selon l'une quelconque des revendications 1 à 4, dans laquelle la première lentille (12) et la seconde lentille (14) comprennent un matériau acrylique mou.

**6.** Lentille selon l'une quelconque des revendications 1 à 4, dans laquelle la seconde lentille (14) comprend un matériau d'hydrogel.

**7.** Lentille selon l'une quelconque des revendications 1 à 4, dans laquelle la seconde lentille (14) comprend un matériau de silicone.

**FIG. 1**

**FIG. 2**

FIG. 3

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6197059 B
- US 5674282 A
- US 5496366 A
- US 5476514 A
- US 6302911 B1, Hanna
- US 6261321 B1

- US 6241777 B1
- US 5275623 A, Sarfarazi
- WO 01134067 A1, Bandhauer
- US 2003204254 A, Alcon Research
- US 7018410 B, Vazeen